# EUROPÄISCHE PATENTANMELDUNG

(11) **EP 2 286 785 A1**
(43) Veröffentlichungstag der Anmeldung: **23.02.2011**
(21) Anmeldenummer: 09168291.4
(22) Anmeldetag: 20.08.2009
(51) Int. Cl.: A61K 8/02, A61K 8/34, A61K 8/97, A61Q 19/10, A61K 36/534, A61Q 19/00

(54) **Zusammensetzung zur Reinigung und/oder Pflege von Haut mit einer ethanolisch-wässrigen hydrodispersiblen Minzkomposition**

(71) Anmelder: Kneipp-Werke Kneipp-Mittel-Zentrale GmbH & Co. KG, 97082 Würzburg (DE)
(72) Erfinder: Wohlfart, Rainer, 97320, Sulzfeld am Main (DE); Ebert, Rebecca, 97852, Schöllbrunn (DE)
(74) Vertreter: Keller, Günter

(57) **Zusammenfassung**

Die vorliegende Erfindung betrifft eine Zusammensetzung zur Reinigung und/oder Pflege von Haut, die eine ethanolisch-wässrige hydrodispersible Minzkomposition in einer Menge von 0,005 - 0,5 Gew.-% bezogen auf das Gesamtgewicht der Zusammensetzung enthält.

## Beschreibung

Gegenstand der vorliegenden Erfindung sind Zusammensetzungen, die zur Behandlung und/oder Pflege von Haut dienen. Durch die Fortschritte der Hygiene nehmen gerade in westlichen Industrieländern Allergien in weiten Teilen der Bevölkerung zu. Aufgrund des wachsenden Wohlstands und der Entwicklung bei der Körperpflege waschen und reinigen sich ein großer Teil der Bevölkerung häufig mehrmals am Tag gründlich und wenden dabei Hautreinigungs- und -pflegemittel, wie Waschlotionen, Hautöle und ähnliches an.

Bei den Produkten zur Reinigung und Pflege der Haut ist wesentlich, dass diese den hygienischen Vorschriften entsprechen und eine ausreichende Zeit der Lagerung ohne Beeinträchtigung (unerwünschte Geruchsveränderungen etc.) überstehen. Andererseits haben die zu diesem Zweck zugefügten Konservierungsmittel, ebenso wie Parfümöle und Duftstoffe die unerwünschte Eigenschaft, dass diese bei häufiger Anwendung der Hautreinigungs- und -pflegemittel von manchen Personen nicht gut vertragen werden, weil entweder schon Unverträglichkeiten hiergegen bestehen oder sich eine Sensibilisierung bis hin zu einer Kontaktallergie durch den häufigen Gebrauch entwickelt. Dies äußert sich meist durch Juckreiz, Rötungen, in schlimmeren Fällen Entzündungen und Hautausschläge, häufig verbunden mit starkem Juckreiz.

Ausgehend davon besteht eine Aufgabe der Erfindung darin, Hautreinigungs- und -pflegemittel bereitzustellen, die einerseits frei sind von Substanzen mit sensibilisierender Wirkung, wie Konservierungsmitteln, Parfümölen, jedoch andererseits (auch ohne Konservierungsmittel) lagerstabil sind und (auch ohne Parfümöle und zum Zweck der Beduftung verwendeter Stoffe) die unangenehmen Eigengerüche von Hilfsstoffen, wie Tensiden oder Emulgatoren überdecken.

Gegenstand der vorliegenden Erfindung ist daher eine Zusammensetzung zur Reinigung und/oder Pflege von Haut, die eine ethanolisch-wässrige hydrodispersible Minzkomposition in einer Menge von 0,005 - 0,5 Gew.-% bezogen auf das Gesamtgewicht der Zusammensetzung enthält.

Einer der Vorteile der Verwendung der ethanolisch-wässrigen hydrodispersiblen Minzkomposition, im folgenden auch wässrige ewhM genannt, liegt darin, dass diese Komponente in einer verhältnismäßig geringen Konzentration zugegeben werden muß. Die ewhM wird bevorzugt in einer Menge zugegeben, die zwischen 0,005 und 0,1 Gew.-%, noch bevorzugter zwischen 0,01 und 0,08 Gew.-% liegt. Der Anteil bezieht sich auf das Verhältnis zwischen Gewicht der zugegebenen Fraktion und Gewicht des fertigen Produktes (Gewicht/Gewicht). Schon bei einem sehr geringen Anteil ewhM haben die Hautreinigungs- und -pflegemittel einen angenehmen Duft und eine kühlende, juckreizlindernde Wirkung.
Unter dem Begriff "hydrodispersibel" wird verstanden, dass die Zusammensetzung in wässriger Phase vollständig gelöst oder dispergiert werden kann. Um die Löslichkeit oder Dispergierbarkeit zu verbessern, kann ein Alkohol, wie Methanol, Propanol oder bevorzugt Ethanol zugesetzt werden.

In einer anderen bevorzugten Ausführungsform wird die ethanolisch-wässrige hydrodispersible Minzkomposition vor der Vermischung mit den anderen Komponenten der Zusammensetzung zuerst mit Ethanol vermischt. Die Menge des Ethanols in der fertigen Zusammensetzung kann 0-20 Gew.-%, bevorzugt 5-15 Gew.-% Ethanol betragen.

Die erfindungsgemäßen Zusammensetzungen zur Reinigung und/oder Pflege von Haut weisen in bevorzugter Ausführungsform keine Parfümöle auf. Unter Parfümölen werden Mischungen unverdünnter ätherischer Öle, natürlicher und/oder synthetischer einheitlicher Duftstoffe und/oder Mischungen dieser Komponenten verstanden, die zum Zweck der Beduftung zugesetzt und so zur Herstellung von Parfüm, Eau de Parfüm, Eau de Toilette oder zur Parfümierung von Kosmetika oder Haushaltsartikeln verwendet werden. Lediglich die ethanolisch-wässrige hydrodispersible Minzkomposition sorgt für einen dezenten, frischen Geruch wobei der bestimmungsgemäße Hauptzweck in der später noch eingehend beschriebenen kühlenden und juckreizlindernden Wirkung liegt. Außerdem weisen die erfindungsgemäßen Zusammensetzungen in bevorzugter Ausführungsform keine weiteren zum Zweck der Beduftung verwendeten Stoffe auf. Bei der ethanolisch-wässrige hydrodispersible Minzkomposition handelt es sich nicht um ein Parfümöl, einen Duftstoff oder ein Konservierungsmittel im Sinne dieser Definition.

In einer besonders bevorzugten Ausführungsform enthalten die erfindungsgemäßen Zusammensetzungen auch eine Derma-Membran-Struktur. Bei der Derma-Membran-Struktur werden vor allem Triglyceride, Cholesterin, Phospholipide, freie Fettsäuren, Squalen und Ceramide eingesetzt. Hierbei handelt es sich um hautverwandte Lipide pflanzlichen Ursprungs, die zur Lipidsubstitution gerade bei geschädigter Hautbarriere besonders geeignet sind. In bevorzugter Ausführungsform weisen die erfindungsgemäßen Zusammensetzungen keine Paraffine und/oder Silikone auf, da diese okklusiv wirkenden Verbindungen den pflegenden Charakter der erfindungsgemäßen Zusammensetzungen eher stören.

Die erfindungsgemäßen Zusammensetzungen sind vor allem für die Anwendung bei solchen Personen geeignet, die Neigungen zu Hautallergien haben oder die unter Erkrankungen der Haut leiden. Derartige Erkrankungen können beispielsweise die Schuppenflechte sein, aber auch trockene Haut und Schädigungen der Haut, die beispielsweise durch berufliche Tätigkeiten, nämlich den häufigen Kontakt mit reizenden Substanzen hervorgerufen werden.

Besonders vorteilhaft bei den erfindungsgemäßen Zusammensetzungen sind die Verringerung des Juckreizes (Antipruritus-Wirkung) und die kühlende Wirkung.

Die ethanolisch-wässrige hydrodispersible Minzkomposition enthält zwar einen Anteil Menthol, das in einer Konzentration ab 0,1 % eine Antipruritus-Wirkung hat (Patri, F.; Silano V.; Plants in Cosmetics, Council of Europe Publishing, Strasbourg (2002). Steinegger, E., Hänsel. R.; Lehrbuch der Pharmakognosie und Phythopharmazie, Springer Verlag Berlin (1988). Die Juckreiz vermindernde Wirkung der ethanolisch-wässrigen hydrodispersiblen Minzkomposition übertrifft die Wirkung des Menthols um ein Vielfaches und setzt bereits ab einer Menge von 0,005 Gew.-% der ewhM (Gewicht/Gewicht) ein. Durch Zusatz einer geringen Ethanolmenge (1-20 %, bevorzugt 2-15 %, besonders bevorzugt 4-12 % Ethanol, bezogen auf die fertige Zusammensetzung) kann dieser Effekt noch intensiviert werden. Für die Antipruritus-Wirkung ist daher nicht oder nicht nur der Mentholbestandteil ausschlaggebend, sondern die anderen Verbindungen, die in der wässrigen Minzölfraktion enthalten sind.

Schon durch die geringen Konzentrationen der ethanolisch-wässrigen hydrodispersiblen Minzkomposition kann der Zusammensetzung ein angenehmer und dauerhaft stabiler Minzduft verliehen werden. Die erfindungsgemäß eingesetzten Konzentrationen der ethanolisch-wässrigen hydrodispersiblen Minzkomposition liegt im Vergleich zu konventionellen ätherischen Ölen und/oder anderen Parfümölen, die üblicherweise Hautpflege- bzw. Hautbehandlungsprodukten zugefügt werden, um etwa eine Zehnerpotenz niedriger.
Aufgrund der geringen Konzentrationen an ewhM treten selbst bei Personen mit empfindlicher Haut keine Unverträglichkeitsreaktionen auf, wie in einer Anwendungsstudie mit Heimapplikation an 75 Probanden/-innen der Universität Osnabrück, Fachbereich Humanwissenschaften, Dermatologie, Umweltmedizin und Gesundheitstheorie bestätigt.

Die Studie wurde an Probanden, 55 weiblich und 20 männlich, 31 ±13 Jahre alt, durchgeführt, die folgende Charakteristika aufwiesen:
- "Empfindliche Haut", d.h. eine anlagebedingte oder erworbene hohe Reaktionsbereitschaft auf Externa (Creme/Lotion/Salbe, Make-up, Wasch-/Pflege-/Reinigungsmittel, etc.) mit Symptomen, wie z.B. Jucken, Brennen, Rötung oder Schuppung.
- "Atopische Diathese", d.h. Neigung zu Heuschnupfen und / oder Neurodermitis

Dabei wurde folgendes Ergebnis erreicht:
- Die Verträglichkeit der Testprodukte ist als "sehr gut" zu beurteilen.
- Das Einziehverhalten der Testprodukte (Pflegeprodukte) ist als "gut" zu beurteilen.
- Das Hautgefühl nach der Anwendung der Testprodukte ist als "gut" zu beurteilen.
- Der Geruch ist als "gut" - "befriedigend" zu beurteilen.

Ein weiterer Vorteil der Zugabe der ethanolisch-wässrigen hydrodispersiblen Minzkomposition ist, dass keine allergenen Stoffe, die im Sinne der Kosmetik-Verordnung deklarationspflichtig sind, zugegeben werden müssen.

Die erfindungsgemäß eingesetzte, ethanolisch-wässrige hydrodispersible Minzkomposition wird vorzugsweise derart hergestellt, dass
1. frisches oder getrocknetes, teilweise zerkleinertes Pflanzenmaterial, nämlich vor allem Blätter, aber auch Stengel von verschiedenen Sorten der Pfefferminzpflanze in ein geeignetes Gefäß verbracht werden und von unten bedampft werden. Der Wasserdampf löst aus den Pflanzenteilen die flüchtigen Bestandteile, vor allem essenzielle Öle heraus. Dieser mit den gewünschten Pflanzenbestandteilen gesättigte Wasserdampf wird über eine geeignete Kühlvorrichtung abgekühlt und in einem geeigneten Gefäß (Tank) abkühlen gelassen. Dabei scheidet sich oben die organische Phase mit den Ölbestandteilen und unten die wässrige Phase ab.
2. die wässrige Phase wird aufkonzentriert
3. eine ethanolische Menthol-Lösung wird zugesetzt. So entsteht die ethanolisch-wässrige hydrodispersible Minzkomposition. Diese zeichnet sich überraschenderweise dadurch aus, dass sie bereits in geringer Menge (0,01 - 0,07%) eine juckreizmildernde und stark kühlende Wirkung entfaltet. Der Vorteil dieser geringen Wirkstoffkonzentration liegt darin dass im Unterschied zu Menthol auch bei hautsensiblen Personen nachweislich keine unangenehmen Hautreaktionen auftreten. Ein erwünschter Nebeneffekt der Komposition ist, der angenehme Geruch, der unangenehme Eigengerüche der Hilfstoffe überdeckt ohne selbst die Definition eines Parfümöls zu erfüllen.

**Tabelle 1: Zusammensetzung der ethanolisch wässrigen hydrodispersiblen Minzkomposition (ewhM)**

| **Chemische Verbindung** | **Anteil der ewhM in Gew.-%** | **Anteil in Gew.-% in bevorzugten Ausführungsformen** |
|---|---|---|
| Ethanol/Ethylalcohol | 10 - 20 | 0,002 - 0,007 |
| Menthol | 20 - 30 | 0,004 - 0,021 |
| wässrige Minzöl-Fraktion | 40 - 50 | 0,008 - 0,035 |

Die gaschromatographische Analyse der wässrigen Minzöl-Fraktion, Tabelle 2, der ewhM, Tabelle 3, und die vergleichende Untersuchung einer Probe ätherischen Pfefferminzöls, Tabelle 4, zeigt quantitative Unterschiede in den Zusammensetzungen. Außerdem fehlen in der wässrigen Minzöl-Fraktion und im ewhM die Komponenten alpha-Pinen und beta-Pinen, die für ein Pfefferminzöl typisch sind.

**Tabelle 2: Zusammensetzung der wässrigen Minzöl-Fraktion (GC-Analyse)**

| **Ingredient** | **Cas** | **Amount** |
|---|---|---|
| Menthone | 14073-97-3 | 26,04% |
| Menthofurane | 494-90-6 | 9,99% |
| Menthyl Acetate | 16409-45-3 | 8,00% |
| Isomenthone | 491-07-6 | 4,50% |
| Pulegone | 3 285-04-9 | 4,12% |
| 1,8 Cineole | 470-85-6 | 3,10% |
| Beta-Caryophyllene | 87-44-5 | 3,03% |
| Terpinen-4-Ol | 562-74-3 | 2,78% |
| Neo-Isomenthol | 491-02-1 | 1,83% |
| Germacrene-D | 37839-63-7 | 1,06% |
| Piperitone | 6091-50-5 | 0,80% |
| Isomenthyl Acetate | 20777-45-1 | 0,48% |
| Beta-Bourbonene | 5208-59-3 | 0,37% |
| Limonene | 5989-27-5 | 0,23% |
| Linalool | 78-70-6 | 0,22% |

**Tabelle 3: Zusammensetzung der ethanolisch-wässrigen hydrodispersiblen Minzölkomposition (GC-Analyse)**

| **Ingredient** | **Cas** | **Amount** |
|---|---|---|
| Menthol | 2216-51-5 | 65,13% |
| Menthone | 14073-97-3 | 9,08% |
| Menthofurane | 494-90-6 | 3,45% |
| Menthyl Acetate | 16409-45-3 | 2,79% |
| Isomenthone | 491-07-6 | 1,57% |
| Pulegone | 3 285-04-9 | 1,46% |
| 1,8 Cineole | 470-85-6 | 1,08% |
| Beta-Caryophyllene | 87-44-5 | 1,06% |
| Terpinen-4-Ol | 562-74-3 | 0,97% |
| Neo-Isomenthol | 491-02-1 | 0,64% |
| Germacrene-D | 37839-63-7 | 0,37% |
| Piperitone | 6091-50-5 | 0,28% |
| Isomenthyl Acetate | 20777-45-1 | 0,17% |
| Beta-Bourbonene | 5208-59-3 | 0,13% |
| Limonene | 5989-27-5 | 0,08% |
| Linalool | 78-70-6 | 0,07% |

**Tabelle 4: Zusammensetzung einer Vergleichsprobe ätherischen Pfefferminzöls (GC-Analyse)**

| **Ingredient** | **Cas** | **Amount** |
|---|---|---|
| Menthol | 2216-51-5 | 47,00% |
| Menthone | 14073-97-3 | 20,50% |
| Menthyl Acetate | 16409-45-3 | 5,00% |
| 1,8 Cineole | 470-85-6 | 4,40% |
| Menthofurane | 494-90-6 | 3,00% |
| Isomenthone | 491-07-6 | 2,90% |
| Pulegone | 3 285-04-9 | 2,20% |
| Beta-Caryophyllene | 87-44-5 | 1,60% |
| Limonene | 5989-27-5 | 1,50% |
| β-Pinen | 127-91-3 | 0,70% |
| Germacrene-D | 37839-63-7 | 0,70% |
| α-Pinen | 7785-70-8 | 0,50% |
| Piperitone | 6091-50-5 | 0,50% |
| P-Cymene | 99-87-6 | 0,40% |
| Sabinene Hydrate | 546-79-2 | 0,40% |
| Sabinene | 3387-41-5 | 0,40% |

Je nach Anwendungsgebiet können die erfindungsgemäßen Zusammensetzungen nach den folgenden bevorzugten Vorschriften hergestellt werden:

### Herstellungsvorschriften

### I) Creme

Eine erfindungsgemäße Creme, die insbesondere für die Anwendung am Gesicht geeignet ist, kann beispielsweise so hergestellt werden, dass zunächst eine Wasserphase hergestellt wird, die neben Wasser und Viskositätsreglern wie Dextrin, Glycerin, gegebenenfalls Mittel zur Einstellung des pH-Wertes wie Citronensäure enthält.

Weiterhin wird die Fettphase, die Öle, Wachse, Sheabutter und weitere Komponenten wie mikrokristalline Zellulose enthält, auf 70-80°C erwärmt und gut gerührt. Dann wird die Wasserphase langsam unter die Fettphase gezogen, wobei eine Mischung der Fett- und der Wasserphase durch Verwendung eines Homogenisators bewirkt werden kann. Die homogenisierte Masse kann dann langsam abgekühlt werden und es wird der pH-Wert auf den gewünschten Bereich eingestellt. Üblicherweise liegt der pH-Wert bei Cremes in dem Bereich zwischen 4,0 und 6,5, bevorzugt 4,5 bis 5,2, besonders bevorzugt bei pH 5,0. Damit das fertige Produkt eine Derma-Membranstruktur aufweist, wird DMS Probiol N 03015 (Kuhs GmbH & Co. Laboratorien, Langfeld, Deutschland) bei einer Temperatur zwischen etwa 25°C und 27°C unter Homogenisierungsbedingungen eingearbeitet.

Anschließend wird die ewhM (Mint Natural Hydrodispersible, Robertet GmbH, Köln, Deutschland), gegebenenfalls vermischt mit Ethanol, zugegeben und anschließend wird die ganze Mischung nochmals homogenisiert. Dann kann die Mischung abgekühlt werden. Es wird eine homogene, gut fließfähige Emulsion erhalten, deren Oberfläche glänzend ist.

### II) Körperlotion

Eine erfindungsgemäße Körperlotion kann beispielsweise so hergestellt werden, dass zunächst eine Wasserphase hergestellt wird, die der Fettphase zugefügt wird.

Dazu wird zuerst Wasser sterilisiert, anschließend auf 60°C abgekühlt. Glycerol 85 %, Dextrin gelöst in Wasser bei 60°C, wasserfreie Citronensäure, ebenfalls gelöst in Wasser bei 60°C, werden unter Rühren bei laufendem Homogenisator (2000U/min) über den Homogenisator eingearbeitet. Bei 70°C wird die Fettphase bestehend aus: Jojobawachs, Sheabutter, Camelinaöl 3/6, Verdickern und Vitaminen, unter Rühren in die Fryma gezogen (Homogenisator bei 2000U/min) und dann homogenisiert bei 3000U/min, 4min über die Ringleitung. Unter Rühren wird auf max. 30°C abgekühlt. Dann wird eine Vormischung aus Ethanol- / Wasser- / ewhM (Mint Natural Hydrodispersible, Robertet GmbH, Köln, Deutschland) und DMS Probiol N 03015 (Kuhs GmbH & Co. Laboratorien, Langfeld, Deutschland) bei Raumtemperatur unter Rühren eingezogen. Anschließend wird über die Ringleitung homogenisiert (2100 U/min).

### III) Hautöl

Zunächst wird ein geeignetes Pflanzenöl, wie beispielsweise Sonnenblumenöl vorgelegt und mit Jojobawachs, Camelinaöl 3/6, Vitaminen und der wässrigen ewhM (Mint Natural Hydrodispersible, Robertet GmbH, Köln, Deutschland) vermischt. Diese Mischung wird gerührt, bis sie klar wird. Dies tritt nach ca. 10-15 Min. ein.

### IV) Waschlotion

Zunächst wird Wasser und Glycerin in einem Mischgefäß vorgelegt. Dann wird Natrium-Cocoyl-Glutamat (Plantacare ACG HC, Cognis GmbH, Monheim am Rhein, Deutschland) zugefügt. Anschließend wird mit der ÖI-IVerdickerphase auf Basis von Jojobawachs vermischt. Die Phasen werden langsam bis zu Homogenität vermischt. Dabei wird die Phase milchig. Anschließend wird die Ethanolphase, bestehend aus Ethanol, wässriger ewhM (Mint Natural Hydrodispersible, Robertet GmbH, Köln, Deutschland) und Glyceryl Caprylate (Dermosoft GMCY, Dr. Straetmans GmbH, Hamburg, Deutschland) eingerührt, bis eine homogene Mischung entsteht. Während der Neutralisationsphase wird Wasser und Citronensäure zuerst gemischt und dann zugeführt. Nach Mischung für 15 -20 Minuten mit dem Homogenisator kann die fertige Lotion entnommen werden.

Die erfindungsgemäßen Zusammensetzungen zur Pflege und/oder Reinigung der menschlichen Haut haben verschiedene überraschende Vorteile. Einerseits können kosmetische Präparate bereitgestellt werden, die frei sind von Parfümölen, und/oder Konservierungsstoffen und lediglich die ethanolisch-wässrige hydrodispersible Minzkomposition in einer sehr geringen Menge aufweisen. Diese Menge liegt bevorzugt zwischen 0,005 und 0,5 Gew.-%, bezogen auf das Gesamtgewicht der Zusammensetzung. Daher sind die erfindungsgemäßen Produkte besonders für Personen mit Hautallergie, trockener Haut und besonders empfindlicher Haut geeignet.

Ein weiterer Vorteil der Zugabe der ethanolisch-wässrigen hydrodispersiblen Minzkomposition liegt darin, dass diese Komponente eine Juckreiz stillende Wirkung aufweist. Das bedeutet, dass durch die Anwendung der erfindungsgemäßen Produkte der Juckreiz gelindert wird. Dies ist vor allem bei Allergikern oder auch bei Personen mit trockener Haut oder mit einschlägigen Erkrankungen, wie Schuppenflechte besonders vorteilhaft. Schließlich vermittelt die erfindungsgemäß eingesetzte ethanolisch-wässrige hydrodispersible Minzkomposition einen dezenten Wohlgeruch, der den unaufdringlichen Eindruck von Frische vermittelt.

Die einzelnen Komponenten der fertigen Produkte variieren hinsichtlich ihrer jeweiligen Bestandteile innerhalb gewisser Grenzen. In der nachfolgenden Tabelle 1 sind die wesentlichen Komponenten der jeweiligen Produkte mit bevorzugten Bereichen angegeben.

**Tabelle 5: Bevorzugte Bereiche der einzelnen Komponenten in den fertigen Produkten**

| | **Creme** | **Körperlotion** | **Hautöl** | **Waschlotion** |
|---|---|---|---|---|
| **Herstellungsbeispiel** | **1** | **2** | **3** | **4** |
| **Wasser** | **35-55** | **55-70** | **0-10** | **45-70** |
| **Pflanzenöl** | **0-10** | **0-10** | **45-65** | **0** |
| **DMS** | **20-50** | **5-20** | **0** | **0-5** |
| **Sheabutter** | **7,5-10** | **2-3** | **0-5** | **0-5** |
| **Ethanol 96 %** | **5-10** | **5-10** | **0-5** | **5-15** |
| **Camelina Öl 3/6** | **2-10** | **2-10** | **5-25** | **1-20** |
| **Jojoba-Wachs** | **2-10** | **2-10** | **25-35** | **4-6** |
| **Sodium Cocoyl Glutamate** | **0** | **0** | **0** | **10-50** |
| **Glycerol 85 %** | **3-10** | **3-10** | **0** | **3-10** |
| **Wässrige Minzöl- Fraktion** | **0,005-0,5** | **0,005-0,5** | **0,005-0,5** | **0,005-0,5** |

Die Mengenangaben in Gewichtsprozent sind bezogen auf das fertige Produkt. Die Angaben ergänzen sich durch übliche Inhaltsstoffe von Kosmetika, wie Mitteln zur Einstellung der Viskosität, wie Xanthan, und Carrageenan, pH-Wert-Regulierern, wie Citronensäure, Vitamine, z.B. Vitamin E in Form von Mischtocopherol 70% (natürlich), etc. zu 100 Gew%.

Die nachfolgenden Beispiele erläutern die vorliegende Erfindung, wobei dem Fachmann bekannt ist, dass derartige Zusammensetzungen ohne Schwierigkeiten hergestellt werden können.

### Beispiel 1

Es wurde eine Creme gemäß dem Herstellungsbeispiel 1 hergestellt.

Der Rezeptur kommt nachweislich eine barriereregenerierende Wirkung zu. Es wurde eine Studie an 21 Personen durchgeführt.

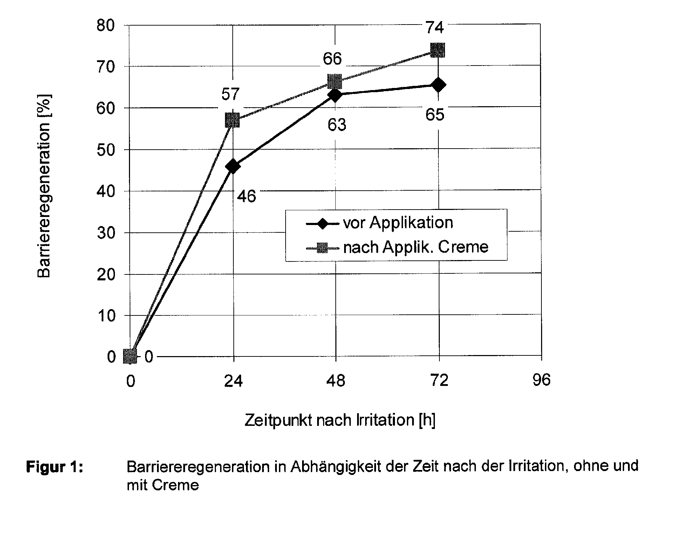

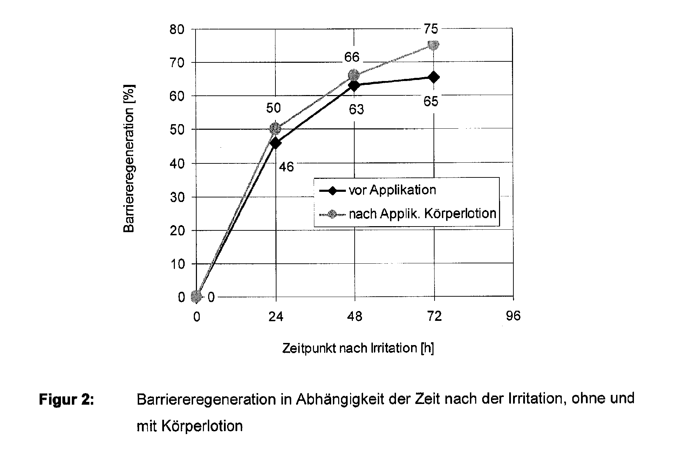

Die Barriereregeneration einer mechanisch geschädigten Haut wird durch die Applikation der Creme gemäß Herstellungsbeispiel 1 unterstützt. Dieser unterstützende Effekt ist bereits 24h nach der Barriereschädigung messbar und verbleibt über die Dauer von 72h nach der Barriereschädigung. Die Ergebnisse sind in Figur 1 dargestellt.

### Beispiel 2

Es wurde eine Körperlotion gemäß Herstellungsbeispiel 2 hergestellt.

Die Barriereregeneration einer zuvor geschädigten Haut wird durch die Applikation der Körperlotion gemäß Beispiel 2 unterstützt. Dieser unterstützende Effekt ist erst 72h nach der Barriereschädigung, dann aber sehr deutlich messbar.

In der Studie wurde für die beiden Testprodukte ein unterstützender Effekt auf die Barriereregeneration nach Schädigung durch Tesafilmabrisse im Vergleich zu einer unbehandelten Kontrolle nachgewiesen.

Für beide Testprodukte gilt: Sie unterstützen die Barriereregeneration der Haut nach mechanischer Schädigung.

## Patentansprüche

1. Parfümölfreie Zusammensetzung zur Reinigung und/oder Pflege von Haut, die eine ethanolisch-wässrige hydrodispersible Minzkomposition in einer Menge von 0,005 - 0,5 Gew.-% bezogen auf das Gesamtgewicht der Zusammensetzung enthält.

2. Zusammensetzung nach Anspruch 1, **dadurch gekennzeichnet, dass** die Zusammensetzung 0,01 - 0,08 Gew.-% einer ethanolisch-wässrigen hydrodispersiblen Minzkomposition bezogen auf das Gesamtgewicht der Zusammensetzung enthält.

3. Zusammensetzung nach einem der vorhergehenden Ansprüche, **dadurch gekennzeichnet, dass** diese keine weiteren zum Zweck der Beduftung verwendeten Stoffe auf und/oder Konservierungsmittel enthält.

4. Zusammensetzung nach einem der vorhergehenden Ansprüche, **dadurch gekennzeichnet, dass** diese maximal 20 Gew.-% Ethanol enthält.

5. Zusammensetzung nach einem der vorhergehenden Ansprüche, **dadurch gekennzeichnet, dass** diese Komponenten enthält, die die Ausbildung einer Derma-Membran-Struktur ermöglichen.

6. Zusammensetzung nach Anspruch 5, **dadurch gekennzeichnet, dass** die Derma-Membran-Struktur durch Zugabe einer oder mehrerer der folgenden Bestandteile: Triglyceride, Cholesterin, Phospholipide, freie Fettsäuren, Sqalen und/oder Ceramide erhalten wird.

7. Zusammensetzung nach einem der Ansprüche 1 bis 6, **dadurch gekennzeichnet, dass** es eine Creme ist.

8. Zusammensetzung nach einem der Ansprüche 1 bis 6, **dadurch gekennzeichnet, dass** es eine Körperlotion ist.

9. Zusammensetzung nach einem der Ansprüche 1 bis 3, **dadurch gekennzeichnet, dass** es eine Waschlotion ist.

10. Zusammensetzung nach einem der Ansprüche 1 bis 3, **dadurch gekennzeichnet, dass** es ein Hautöl ist.

11. Zusammensetzung nach einem der vorhergehenden Ansprüche, **dadurch gekennzeichnet, dass** diese für die Anwendung bei Personen mit Neigung zu hochsensibler/trockener Haut oder Hautallergien geeignet ist.

12. Verwendung einer ethanolisch-wässrigen hydrodispersiblen Minzkomposition zur Herstellung einer Zusammensetzung zur Reinigung und/oder Pflege der Haut, die insbesondere für an trockener Haut und Hautallergien leidende Menschen geeignet ist.
